(19) Europäisches Patentamt
European Patent Office
Office européen des brevets

(11) Veröffentlichungsnummer: **0 109 360**
**B1**

(12) # EUROPÄISCHE PATENTSCHRIFT

(45) Veröffentlichungstag der Patentschrift: **13.08.86**

(21) Anmeldenummer: **83810510.4**

(22) Anmeldetag: **07.11.83**

(51) Int. Cl.⁴: **C 07 D 345/00**, C 25 B 3/02, C 07 C 65/34, C 07 C 51/083, C 07 C 50/24, C 07 C 25/22, H 01 B 1/12, H 01 G 4/14, H 01 M 4/60, G 11 B 7/00, G 03 C 1/72

(54) **Metallisch leitendes (2-Fluor-5,6,11,12-tetraselenotetracen)2-Bromid, Verfahren zu dessen Herstellung und dessen Verwendung.**

(30) Priorität: **12.11.82 CH 6611/82**

(43) Veröffentlichungstag der Anmeldung: **23.05.84 Patentblatt 84/21**

(45) Bekanntmachung des Hinweises auf die Patenterteilung: **13.08.86 Patentblatt 86/33**

(84) Benannte Vertragsstaaten: **BE CH DE FR GB LI NL**

(56) Entgegenhaltungen:
**EP-A-0 023 988**
**DE-A-2 535 124**
**DE-A-2 641 742**
**US-A-4 036 648**
**US-A-4 046 950**

**HELVETICA CHIMICA ACTA, Band 61, 1978, Nr. 146, Basel, B. HILTI et al. "Electrical properties and structure of the organic metallic compound bis (Tetraselenotetracene)-Ioide, (TSeT)2-I", Seiten 1462-1469**

(73) Patentinhaber: **CIBA- GEIGY AG, Klybeckstrasse 141, CH- 4002 Basel (CH)**

(72) Erfinder: **Hilti, Bruno, Dr., Marschalkenstrasse 27, CH- 4054 Basel (CH)**
Erfinder: **Mayer, Carl W., Dr., Niederholzboden 55, CH- 4125 Riehen (CH)**
Erfinder: **Rihs, Grety, Birsfelderstrasse 51, CH- 4132 Muttenz (CH)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft das (2-Fluor-5, 6, 11, 12-tetraselenotetracen)$_2$-Bromid als neue metallisch leitende Verbindung, Verfahren zu dessen Herstellung und dessen Verwendung.

Aus der Literatur sind verschiedene metallisch leitende chalkogenierte Tetracen-Komplexe, wie das (5, 6, 11, 12-Tetraselenotetracen)$_2$-Jodid, -Bromid oder -Chlorid oder der (5, 6, 11, 12-Tetrathiotetracen)$_2$-(Jod)$_3$-Komplex bekannt. Diese Komplexe zeigen bei Temperaturen zwischen etwa 30 und 45°K einen relativ scharfen Uebergang vom metallischen in den nicht-leitenden Zustand, d.h. die metallische Phase dieser Komplexe ist nicht bis zu genügend tiefen Temperaturen, bei denen z.B. Supraleitung erwartet werden kann, stabil. Es ist auch bekannt, dass der Uebergangspunkt vom metallischen zum nicht-leitenden Zustand bei dem (Tetrathiotetracen)$_2$-(Jod)$_3$-Komplex unter Druck oder durch Veränderung der Stöchiometrie (Erhöhung der Jod-Konzentration über das Verhältnis 2:3 hinaus) erniedrigt werden kann. Es wird angenommen, dass die Stabilisierung der metallischen Phase bei von der exakten 2:3 Stöchiometrie abweichenden Komplexen durch eine Aenderung der Bandfüllung hervorgerufen wird. Der Mechanismus, welcher unter Einwirkung von Druck beiden obigen Komplexen die Stabilisierung der metallischen Phase bewirkt, ist noch weitgehend unbekannt [vgl. z.B. Chemica Scripta, 17, 23 (1981), deutsche Patentschrift 26 41 742, Helv.Chim. Acta, 61, 1462 (1978) und Extended Linear Chain Compounds, Verlag J.S. Miller, Plenum Press, New York, 385 (1982)].

Aufgabe der Erfindung ist die Bereitstellung einer neuen Verbindung aus der Klasse der chalkogenierten polycyclischen Aromaten, deren metallische Phase bis zu sehr tiefen Temperaturen stabil ist, um damit mit der metallischen Phase in das Temperaturgebiet möglicher Supraleitung vorzudringen.

Es wurde nun gefunden, dass sich der Komplex der Formel I

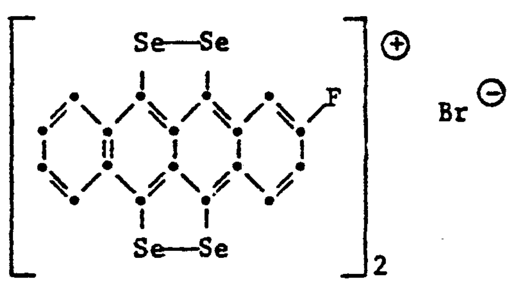

(I)

bei Normaldruck überraschenderweise und im Gegensatz z.B. zu den vorerwähnten (Tetraselenotetracen)$_2$-Jod-, -Brom- oder -Chlorkomplexen durch eine stabile metallische Phase bis mindestens 5°K auszeichnet, d.h. die elektrische Leitfahigkeit des Komplexes nimmt von Raumtemperatur (20-25°C) bis mindestens 5°K (= -268°C) stetig zu. Bei Raumtemperatur weist der Komplex der Formel 1 eine elektrische Leitfähigkeit [$\sigma$] von bis zu 2000 ohm$^{-1}$cm$^{-1}$ und bei 5°K eine solche von 6000 ohm$^{-1}$cm$^{-1}$ auf (gemessen längs der bevorzugten Wachstumsrichtung = Nadelachse).

Der erfindungsgemässe Komplex weist die Raumgruppe $P2_12_12_1$

auf. Die Längen der Achsen der Elementarzelle sind: a = 17,655 Å, b = 17,661 Å, c = 5,125 Å. Der Komplex ist orthorhombisch und nicht-zentrosymmetrisch und weist neben der hohen elektrischen Leitfähigkeit auch eine ausgeprägte elektrische und optische Anisotropie auf.

Der erfindungsgemässe Komplex kann z.B. in Form von mikrokristallinen Pulvern, als amorphe Schicht, als Schicht aus Mikrokristallen, als amorphes Pulver oder in Form von Einkristallen vorliegen.

Figur 1 zeigt eine Projektion der Kristallstruktur des erfindungsgemässen Komplexes;

Figur 2 illustriert die Temperaturabhängigkeit des elektrischen Widerstandes des erfindungsgemässen Komplexes.

Der Komplex der Formel I kann nach verschiedenen Methoden hergestellt werden, z.B. durch (direkte) Oxidation von 2-Fluor-5, 6, 11, 12-tetraselenotetracen mit Brom oder einem oxidierenden, Bromid abspaltenden Bromsalz, wie Kupfer(11)bromid und $FeBr_3$, in Gegenwart eines inerten organischen Lösungsmittels. Geeignete inerte organische Lösungsmittel sind z.B. halogenierte aliphatische Kohlenwasserstoffe, wie Methylenchlorid und 1, 1, 2-Trichloräthan; polare substituierte, besonders halogenierte aromatische Kohlenwasserstoffe, wie Chlorbenzol, o-Dichlorbenzol, 1, 2, 3-Trichlorbenzol, 1, 2, 4-Trichlorbenzol und chlorierte Naphthaline; andere polare Lösungsmittel, wie Benzonitril und Alkylnitrile mit 2-5 C-Atomen, z.B. Acetonitril, Propionitril und Butyronitril; Nitrobenzol; N, N-Dialkylamide von aliphatischen Monocarbonsäuren mit 1-4 C-Atomen im Säureteil, z.B. N, N-Dimethylformamid und N, N-Dimethylacetamid; N, N, N', N'-Tetramethylharnstoff; Dialkylsulfoxide, wie Dimethylsulfoxid und Diäthylsulfoxid; cyclische Aether, wie Tetrahydropyran, Tetrahydrofuran und Dioxan. Es können auch Gemische der genannten Lösungsmittel eingesetzt werden. Die Reaktionstemperaturen bei diesen Oxidationsreaktionen liegen im allgemeinen zwischen 20 und 120°C.

Der Komplex der Formel 1 kann auch durch Diffusion von Brom aus der Gasphase oder aus einer Trägerlösung

in eine Lösung des 2-Fluor-5, 6, 11, 12-tetraselenotetracens hergestellt werden, wobei als Lösungsmittel solche der oben angegebenen Art in Betracht kommen.

Der Komplex der Formel 1 kann ferner aus der Gasphase, d.h. durch Co-Sublimation von 2-Fluor-5, 6, 11, 12-tetraselenotetracen und Brom analog dem in der deutschen Patentschrift 26 41 742 beschriebenen Verfahren hergestellt werden. Dabei lässt man das 2-Fluor-5, 6, 11, 12-tetraselenotetracen und das Brom zweckmässig in einer Inertgasatmosphäre miteinander reagieren, bevorzugt in offenem System. Die Umsetzung in der Gasphase kann aber auch in geschlossenem System in Inertgasatmosphäre vorgenommen werden. Die Umsetzung in der Gasphase kann beispielsweise so erfolgen, dass man Bromdampf mittels eines inerten Trägergases mit 2-Fluor-5, 6, 11, 12-tetraselenotetracen in der Gasphase bei ca. 260° C in Kontakt bringt. Die Kristalle wachsen dabei an den Reaktorwändenund/oder aufeinem gegebenenfalls im Reaktor angeordneten Substrat, wie Aluminiumoxid oder bevorzugt Quarz, in beliebiger Form, z.B. in Form von Stäben oder Rohren. Als Trägergase verwendet man bei dieser Herstellungsmethode zweckmässig hochreine Inertgase, wie Argon, Stickstoff, Helium und Xenon. Die Reaktionstemperaturen bei der Gasphasenumsetzung liegen zweckmässig zwischen 180 und 300° C. Die durch Co-Sublimation erhaltenen Kristalle lassen sich leicht aus der Reaktionskammer bzw. vom Substrat entfernen. Eine geeignete Versuchsanordnung für diese Herstellungsmethode ist in der oben erwähnten deutschen Patentschrift 26 41 742 beschrieben.

Bevorzugt wird der erfindungsgemässe Komplex jedoch durch elektrochemische Oxidation des 2-Fluor-5,6,11,12-tetraselenotetracens in Gegenwart eines inerten organischen Lösungsmittels und eines bromidhaltigen Leitelektrolyten hergestellt. Als inerte organische Lösungsmittel können solche der oben angegebenen Art eingesetzt werden. Bevorzugt sind cyclische Aether und N,N-Dialkylamide von aliphatischen Monocarbonsäuren oder Gemische davon, besonders Tetrahydrofuran und N,N-Dimethylformamid oder Gemische davon. Geeignete bromidhaltige Leitelektrolyte sind z.B. Salze der Formel II

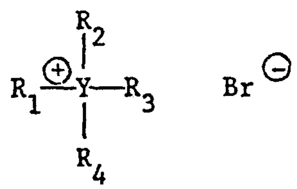 (II),

worin Y N, P oder As und $R_1$ bis $R_4$ unabhängig voneinander $C_{1\ 18}$-Alykl, Benzyl, Phenyl oder Naphthyl darstellen. Alkylgruppen $R_1$ bis $R_4$ können geradkettig oder verzweigt sein und weisen bevorzugt 1-12 C-Atome auf. Als Beispiele solcher Alkylgruppen seien genannt: Methyl, Aethyl, n-Propyl, Isopropyl, n-Butyl, sek-Butyl, tert-Butyl, 1, 1, 3, 3-Tetramethylbutyl, n-Pentyl, 2-Pentyl, n-Hexyl, n-Heptyl, n-Octyl, n-Decyl, n-Dodecyl, n-Tetradecyl, n-Hexadecyl und n-Octadecyl. Bevorzugt verwendet man Verbindungen der Formel II, worin Y N oder P, $R_1$ Benzyl oder Phenyl und $R_2$ bis $R_4$ geradkettiges Alkyl mit je 1-12 C-Atomen oder Phenyl oder $R_1$ bis $R_4$ geradkettiges Alkyl nit je 1-12 C-Atomen bedeuten. Besonders bevorzugt verwendet man Verbindungen der Formel II, worin Y N und $R_1$ bis $R_4$ geradkettiges Alkyl mit je 1-6 C-Atomen und insbesondere je n-Butyl bedeuten.

Je nach Temperatur der Elektrolysezelle und eingesetzten Lösungsmittel verwendet man zweckmässig zwischen 0,01 bis 100 g Leitelektrolyt pro Liter. Als Elektrolysezellen können an sich bekannte Vorrichtungen verwendet werden, z.B. solche, worin der Anodenraum durch Teflonsiebe, Glasfritten oder Kapillaren vom Kathodenraum getrennt ist. Die Dimensionen der Elektrolysezellen können in Abhängigkeit der verwendeten Menge an Reaktionskomponenten variieren, beeinträchtigen jedoch die Qualität des erhaltenen Komplexes der Formel I praktisch nicht. Für die Herstellung von etwa 5-50 mg Komplex der Formel I sind z.B. Zellvolumen von 15-100 ml besonders geeignet.

Die Reaktionstemperaturen (Temperaturen der Elektrolysezellen) liegen je nach Art des verwendeten Lösungsmittels mit Vorteil zwischen 0 und 120° C. Die Stromstärke variiert im algemeinen zwischen 0,005 µA und 5 µA. Der Durchmesser der Anoden und Kathoden beträgt zweckmässig zwischen 0,1 bis 5 mm.

Bei den obigen Umsetzungen werden das 2-Fluor-5, 6, 11, 12-tetraselenotetracen und das Brom oder Bromidsalz in mindestens stöchiometrischer Menge eingesetzt. Im allgemeinen empfiehlt es sich jedoch, mit einem Ueberschuss an Brom oder Bromidsalz zu beginnen, so dass sich in der Reaktionsphase zu jeder Zeit ein 20- bis 1000-facher molarer Ueberschuss an Brom oder Bromidsalz befindet.

Gegenstand der Erfindung sind auch die zur Herstellung des Komplexes der Formel I entwickelten neuen Zwischenprodukte der Formeln III bis VI

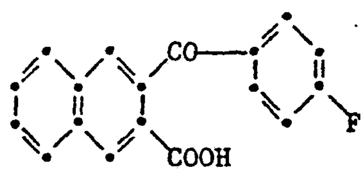

(III),

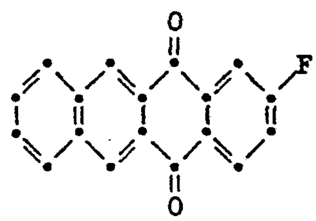

(IV),

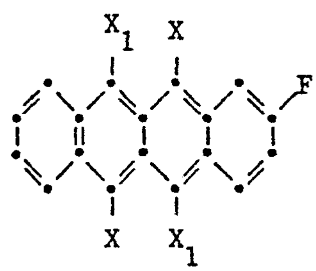

(V) und

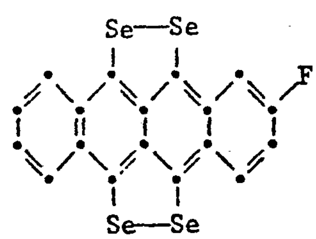

(VI),

worin die $X_1$ und X je Wasserstoff oder die $X_1$ je Wasserstoff und die X je Chlor oder die $X_1$ je Chlor und die X je Wasserstoff bedeuten.

Die Verbindungen der Formeln III bis VI können in Analogie zu an sich bekannten Verfahren dadurch hergestellt werden, indem man 2, 3-Naphthalindicarbonsäureanhydrid in Gegenwart eines Friedel-Crafts-Katalysators, bevorzugt Aluminiumchlorid, mit Fluorbenzol zu einer Verbindung der Formel III umsetzt, die 2-(4-Fluorbenzoyl)-naphthalin-3-carbonsäure zum 2-Fluor-5, 12-naphthacenchinon (Verbindung der Formel IV) cyclisiert, die Verbindung der Formel IV zum 2-Fluor-tetracen (Verbindung der Formel V mit $X_1$ und X = H) reduziert, z.B. in Gegenwart von Zinkstaub in saurem Medium, wie z.B. Essigsäure, das 2-Fluortetracen mit Sulfurylchlorid zum 2-Fluor-5, 11- bzw. 2-Fluor-6, 12-dichlortetracen umsetzt [vgl. z.B. Bull. Soc. Ghim. France, 427 (1948)], und schliesslich das 2-Fluor-5, 11- bzw. 2-Fluor-6, 12-dichlortetracen oder deren Gemisch bei erhöhter Temperatur mit Selen umsetzt.

Die Cyclisierung der 2-(4-Fluorbenzoyl)-naphthalin-3-carbonsäure zum 2-Fluor-5, 12-naphthacenchinon kann in ansich bekannter Weise in Gegenwart einer Protonensäure oder einer Lewis-Säure vorgenommen werden. Beispiele geeigneter Protonensäuren sind Polyphosphorsäure, Chlorsulfonsäure und Schwefelsäure. Als Lewis-Säuren kommen z.B. Bortrifluorid und vor allem Aluminiumtrichlorid in Betracht. Bevorzugt ist die Cyclisierung in Gegenwart einer Lewis-Säure, besonders Aluminiumchlorid, in der Schmelze. Die Umsetzung des 2-Fluortetracens mit dem Sulfurylchlorid sowie die Umsetzung des 2-Fluor-5, 11- bzw. 2-Fluor-6, 12-dichlortetracens mit dem Selen werden zweckmässig in Gegenwart eines inerten organischen Lösungsmittels vorgenommen. Für die Umsetzung des 2-Fluortetracens mit dem Sulfurylchlorid eignen sich z.B. Nitrobenzol,

Benzol und Tetrachlorkohlenstoff. Bevorzugtes Lösungsmittel ist Nitrobenzol.

Die Umsetzung des 2-Fluor-5, 11- bzw. 2-Fluor-6, 12-dichlortetracens wird bevorzugt in Gegenwart eines halogenierten aromatischen Kohlenwasserstoffs, insbesondere Trichlorbenzol, vorgenommen.

Auf Grund der metallisch elektrischen Leitfähigkeit und der ausgeprägten elektrischen und optischen Anisotropie eignet sich der erfindungsgemässe Komplex zur Verwendung als organisches Leiterelement z.B. für leitende Beschichtungen auf Kunststoffasern; des weiteren als Polarisatormaterial oder als Zusatz zu antistatischen Beschichtungen und Belägen, beispielsweise solchen auf Kunststoff-Basis. Der Komplex der Formel I kann auch in hochleitenden, elektronenstrahl- oder photonenempfindlichen Druckmaterialien oder -verfahren eingesetzt werden, wie sie z.B. in der europäischen Patentanmeldung Veröff. Nr. 23 988 und der U.S. Patenschrift 4.036.648 beschrieben sind. Dank seiner Redoxeigenschaften und der verschiedenem intensiven Farben seiner Redoxstufen (blaugrün, grün, blau, gelb) kann der Komplex der Formel I ferner mit Vorteil im Informationssystemen, wie Farbbildschirmen, sowie in elektronischen Komponenten verwendet werden. Der hochleitende Komplex der Formel 1 ist hierzu besonders geeignet, da er weiterer Oxidation und Reduktion in elektrischen Anordnungen, wie sie z.B. elektrochrome Schaltungen darstellen, unterworfen werden kann. Vor allem eignet sich der erfindungsgemässe Komplexe jedoch auf Grund seiner bis zu ca. 5°K stabilen metallischen Phase für verschiedene Anwendungen in der Tieftemperaturtechnologie, z.B. zur Verwendung in Kondensatorfolien oder aktiven Batterieelektroden, deren Gebrauch damit auch bei tiefen Temperatur möglich ist.

**Beispiel**

a) 45,0 g (227 mMol) 2, 3-Naphthalindicarbonsäureanhydrid werden in 200 ml (2, 1 Mol) Fluorbenzol suspendiert. Innerhalb von 5 Minuten werden unter starkem Rühren 75,5 g (565 mMol) pulverisiertes Aluminiumchlorid zugegeben (exotherme Reaktion bis ca. 32°C). Die dunkelrote Suspension wird 6 Stunden am Rückfluss erhitzt und gerührt.

Dann lässt man die Lösung auf Raumtemperatur abkühlen, giesst sie auf ca. 500 g Eis und rührt bis zur Vervollständigung der Hydrolyse. Ueberschüssiges Fluorbenzol wird abgedampft, und das erhaltene Produkt wird in Wasser suspendiert. Die Suspension wird abfiltriert, das Filtrat mit Wasser nachgewaschen und das Produkt getrocknet. Man erhält 76 g rohe 2-(4-Fluorbenzoyl)-naphthalin-3-carbonsäure. Das Rohprodukt wird mit 2 Liter 10%iger Sodalösung verrührt, die erhaltene Lösung wird mit Salzsäure angesäuert, und der weisse Niederschlag wird abfiltriert und getrocknet. Man erhält 54,2 g (81% d.Th.) 2-(4-Fluorbenzoyl)-naphthalin-3-carbonsäure.

MS: (M+ = 294; M+-COOH = 249, M+-COO = 250, M+-$C_6H_4F$ = 199); IR-Spektrum (KBr): OH ca. 3300 cm; G=O-Doppelbande 1695/1705 $cm^{-1}$.

b) 200 g (1,5 Mol) pulverisiertes Aluminiumchlorid und 40 g (684 mMol) Natriumchlorid werden zusammen auf 140-150°C erhitzt. Nach etwa 1,5 Stunden werden 40 g (136 mMol) 2-(4-Fluorbenzoyl)-naphthalin-3-carbon-säure zur Schmelze gegeben. Das dunkelrote Gemisch wird eine Stunde bei 140-150°C gerührt. Anschliessend wird bei etwa 100°C durch langsame Zugabe von Eiswasser hydrolysiert. Der Niederschlag wird abfiltriert und mit 10%iger Sodalösung verrührt. Dann wird mit Wasser neutral gewaschen und getrocknet. Das erhaltene Produkt wird bei 230°C sublimiert. Man erhält 13,1 g (70% d.Th.) 2-Fluor-5, 12-naphtha-cenchinon.

DC: Kieselgel, Benzol: $R_x \cong 0,6$; gelb fluoreszierender Fleck.

NMR (100 MHz in $CDCl_3$): komplizierte, aber interpretierbare Multipletts im Aromatenbereich.

MS: M+ = 276, M+-CO = 248, M+-2G0 = 220.

c) 8,0 g (29 mMol) 2-Fluor-5, 12-naphthacenchinon, 40 ml Wasser, 680 ml Essigsäure und 40 g (611 mMol) Zinkstaub werden zusammengegeben und zum Rückfluss erhitzt. Nach 30 Minuten Rühren am Rückfluss wird abgekühlt, und es werden 200 ml Wasser zugegeben. Die entstandene Suspension des 2-Fluortetracens wird abdekantiert, wobei der Zinkstaub im Kolben zurückbleibt. Das 2-Fluortctracen wird abfiltriert, mit Wasser und Aethanol gewaschen und getrocknet. Nach dem Umkristallisieren aus 500 ml Xylol erhält man 4,0 g (56% d.Th.) 2-Fluortetracen.

UV (Benzol): typisches Tetracenspektrum. $\lambda_{max}$ 476, 446, 420, 394 nm.

MS: M+ = 246, $M^{+2}$ = 123.

d) 5,0 g (20,3 mMol) 2-Fluortetracen werden unter Stickstoff in 25 ml Nitrobenzol suspendiert und auf 5°C abgekühlt. Dann lässt man innerhalb von 30 Minuten 5,9 g (43,7 mMol) Sulfurylchlorid in 25 ml Nitrobenzol zutropfen und rührt 2 Stunden bei 5°C. Dann lässt man die Temperatur auf 20-25°C ansteigen, heizt innerhalb von 1,5 Stunden auf 90°C, rührt 10 Minuten bei dieser Temperatur und kühlt ab. Die Suspension wird abfiltriert, mit ca. 600 ml Aethanol nachgewaschen und getrocknet. Man erhält 5,0 (78% d.Th.) 2-Fluor-5, 11- bzw. 6, 12-dichlortetracen.

MS: M+ = 314/316 (= 2 Cl), M+-HCl = 278, M+-2C1 = 244, $M^{+2}$ = 157/158 (2 Cl).

e) 7,2 g (22,8 mMol) 2-Fluor-5,11-dichlortetracen, 7,7 g (97,5 mMol) Selen und 175 ml Trichlorbenzol werden zusammengegeben und 120 Stunden bei einer Badtemperatur von 250°C unter Stickstoff am Rückfluss gekocht. Nach 70 Stunden gibt man nochmals 3,8 g (48,1 mMol) Selen zu.Dann lässt man die Suspension abkühlen, verdünnt mit ca. 200 ml n-Hexan und filtriert ab. Das Produkt wird mit Benzol und n-Hexan nachgewaschen und getrocknet und anschliessend am Hochvakuum bei 260-270°C/10 $^{-3}$ barsublimiert. Man erhält 4,4 g (35% d.Th.) 2-Fluor-5, 6, 11, 12-tetraselenotetracen.

UV-Spektrum in Trichlorbenzol$\lambda_{max}$ = 719, 659, 466 nm.

MS (bei 260°C/$10^{-3}$bar sublimiert): $M^+$ = 560 (Cluster 4 Se), $M^+$-Se = 480 (Cluster 3 Se), $M^+$-2Se = 400 (Cluster 2 Se), $M^+$-3Se = 322/320, $M^{+2}$ = 279 (Cluster 4 Se), $M^+$-$C_{18}H_7F$ = 242. Kristallstruktur: Raumgruppe

$P2_12_12_1$;

Achsen a = 21 538 Å, b = 17,351 Å, c = 4,033 Å.

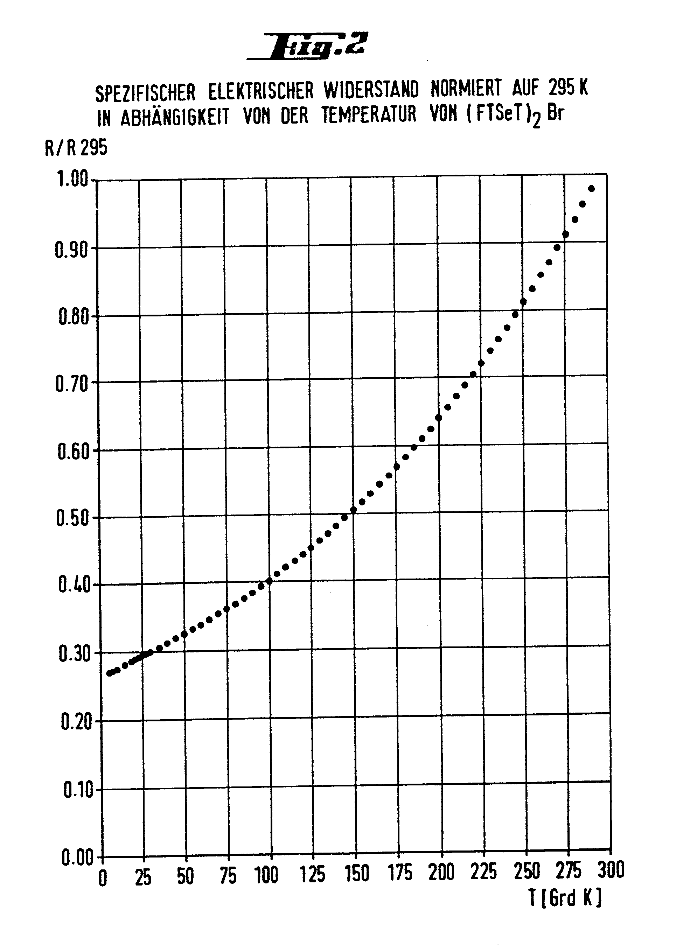

f) 30 mg (2-Fluor-5, 6, 11, 12-tetraselenotetracen)$_2$ werden in den Anodenraum einer Elektrolysenzelle mit einem Volumen von 40 ml eingefüllt. Als Elektrolyt werden 200 mg Tetra-n-butyl-ammoniumbromid zugesetzt. Die Zelle wird über Nacht in einem Trockenschrank bei 5 x$10^{-2}$ mbar evakuiert und mit Argon gespult. Dann werden als Lösungsmittel 33 ml eines Gemisches aus 90 Vo1.% Tetrahydrofuran und 10 Vo1.% N, N-Dimethylformamid zugegeben. Nach 12 Stunden Aufheizen auf 60° C wird die Zelle unter 0,6 Volt Spannung gesetzt, wobei sich ein Elektrolysestrom von 1 µA einstellt. Nach 21 Tagen werden die an der Anode (Durchmesser 1 mm; 80 Gew.% Pt, 20 Gew.% Ir) gebildeten Kristalle durch Abwaschen mit Aethanol abgelöst. Sechs Kristalle mit den durchschnittlichen Abmessungen 6 mn x 50 µ x 50 µ werden mittels Platinpaste (Pt-Paste 308 A der Fa. Degussa) auf 4 Sonden montiert, die aus 25 µ dicken Golddrähten bestehen. Die Leitfähigkeit der Kristalle bei Raumtemperatur, gemessen in der obigen Sondenanordnung variiert von 1000 bis 2000 $ohm^{-1}cm^{-1}$. Die Temperaturabhangigkeit des spezifischen Widerstandes, normiert auf 295°K, zeigt für alle Kristalle innerhalb einer Messgenauigkeit von 2% das auf Figur 2 dargestellte Verhalten.

## Patentansprüche

1. Komplex der Formel I

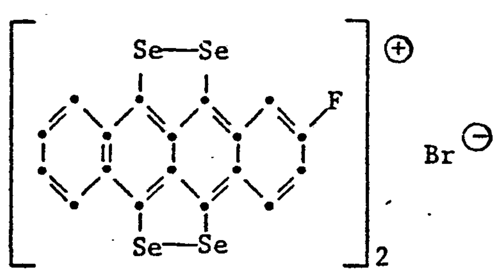

(I).

2. Verfahren zur Herstellung des Komplexes der Formel I nach Anspruch 1, durch Oxidation von 2-Fluor-5, 6, 11, 12-tetraselenotetracen mit Brom oder einem oxidierenden, Bromid abspaltenden Bromsalz, in Gegenwart eines inerten organischen Lösungsmittels.

3. Verfahren zur Herstellung des Komplexes der Formel I nach Anspruch 1, durch Co-Sublimation von 2-Fluor-5, 6, 11, 12-tetraseleno-tetracen und Brom.

4. Verfahren zur Herstellung des Komplexes der Formel I nach Anspruch 1, durch elektrochemische Oxidation von 2-Fluor-5, 6, 11, 12-tetraselenotetracen in Gegenwart eines inerten organischen Lösungsmittels und eines bromidhaltigen Leitelektrolyten.

5. Verfahren nach Anspruch 4, dadurch gekennzeichnet, dass man als, Leitelektrolyten eine Verbindung der Formel II

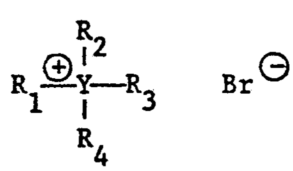

(II)

verwendet, worin

Y N, P oder As und $R_1$ bis $R_3$ unabhängig voneinander $C^{1-18}$-Alkyl, Benzyl, Phenyl oder Naphthyl darstellen.

6. Verfahren nach Anspruch 5, dadurch gekennzeichnet, dass man eine Verbindung der Formel II verwendet, worin Y N oder P, $R_1$ Benzyl, oder Phenyl und $R_2$ bis $R_4$ geradkettiges Alkyl mit je 1-12 C-Atomen, oder Phenyl

oder $R_1$ bis $R_4$ geradkettiges Alkyl mit je 1-12 C-Atomen darstellen.

7. Verfahren nach Anspruch 5, dadurch gekennzeichnet, dass man eine Verbindung der Formel II verwendet, worin Y N und $R_1$ bis $R_4$ geradkettiges Alkyl mit je I-6 C-Atomen, besonders je n-Butyl, darstellen.

8. Verbindungen der Formeln III bis VI

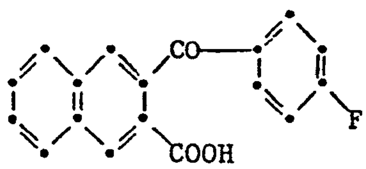

(III),

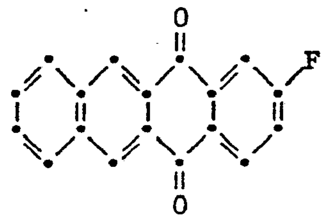

(IV),

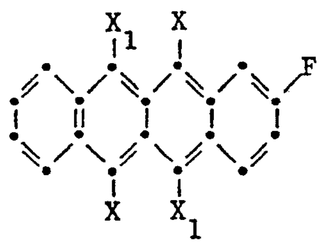

(V) . und

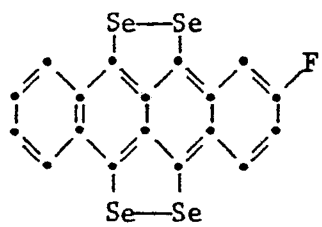

(VI),

worin die $X_1$ und X je Wasserstoff oder die $X_1$ je Wasserstoff und die X je Chlor oder die $X_1$ je Chlor und die X je Wasserstoff bedeuten.

9. Verfahren zur Herstellung der Verbindungen der Formeln III bis VI nach Anspruch 8, dadurch gekennzeichnet, dass man 2, 3-Naphthalindi-carbonsäureanhydrid in Gegenwart eines Friedel-Crafts-Katalysators mit Fluorbenzol zur Verbindung der Formel III umsetzt, die 2-(4-Fluor-benzoyl)-naphthalin-3-carbonsäure zum 2-Fluor-5, 12-naphthacenchinon (Verbindung der Formel IV) cyclisiert, die Verbindung der Formel IV zum 2-Fluortetracen (Verbindung der Formel V mit $X_1$ und X = H) reduziert, das 2-Fluortetracen mit Sulfurylchlorid zum 2-Fluor-5, 11- bzw. 2-Fluor-6, 12-dichlortetracen umsetzt und anschliessend das 2-Fluor-5, 11-bzw. 2-Fluor-6, 12-dichlortetracen oder deren Genisch bei erhöhter Temperatur mit Selen zum 2-Fluor-5, 6, 11, 12-tetraselenotetracen umsetzt.

10. Verwendung der Verbindung der Formel I nach Anspruch 1 als organisches Leiterelement.

**Claims**

1. A complex of the formula I

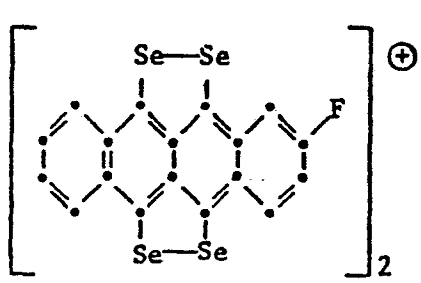

$Br^{\ominus}$ (I).

2. A process for producing the complex of the formula I according to claim 1 by oxidation of 2-fluoro-5, 6, 11 12-tetraselenotetracene with bromine or with an oxidising bromide salt which releases bromine, in the presence of an inert organic solvent.

3. A process for producing the complex of the formula I according to claim 1 by co-sublimation of 2-fluoro-5, 6,

11, 12-tetraselenotetracene and bromine.

4. A process for producing the complex of the formula I according to claim 1 by electrochemical oxidation of 2-fluoro-5, 6, 11, 12-tetraselenotetracene in the presence of an inert organic solvent and a bromide-containing electrolyte.

5. A process according to claim 4, wherein the electrolyte is a compound of the formula II

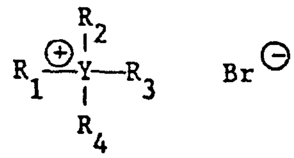

(II)

wherein Y is N, P or As, and each of $R_1$to $R_4$ independently of one another is $C_1$-$C_{18}$alkyl, benzyl, phenyl or naphthyl.

6. A process according to claim 5, which comprises the use of a compound of the formula II wherein y is N or P, $R_1$ is benzyl or Phenyl, and each of $R_2$ to $R_4$ is straight chain alkyl having l-12 C atoms or phenyl, or each of $R_1$ to $R_4$ is straight chain alkyl having 1-12 C atoms.

7. A process according to claim 5, which comprises the use of a compound of the formula II wherein Y is N, and each of $R_1$ to $R_4$ is straight chain alkyl having 1-6 C atoms, particularly n-butyl.

8. Compounds of the formulae III to VI

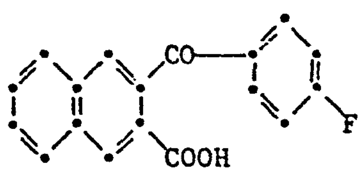

(III),

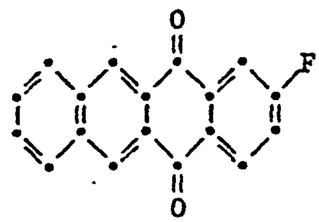

(IV),

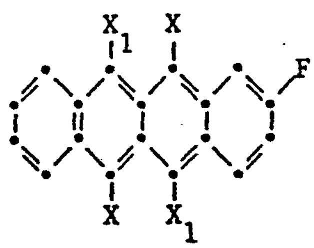

(V)

and

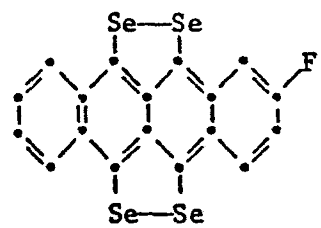

(VI),

wherein each of the symbols $X_1$ and X is hydrogen, or each $X_1$ is hydrogen and each X is chlorine, or each $X_1$ is chlorine and each X is hydrogen.

9. A process for producing the compounds of the formulae III to VI according to claim 8, which process comprises reacting 2, 3-naphthalenedicarboxylic anhydride, in the presence of a Friedel-Crafts catalyst, with fluorobenzene to a compound of the formula III. cyclising the 2-(4-fluoro-benzoyl)naphthalene-3-carboxylic acid to 2-fluoro-5, 12-naphthacenequinone (compound of the formula IV); reducing the compound of the formula IV to 2-fluorotetracene (compound of the formula V wherein $X_1$ and X = H); reacting the 2-fluorotetracene with sulfuryl chloride to 2-fluoro-5, 11- or 2-fluoro-6, 12-dichlorotetracene; and finally reacting the 2-fluoro-5, 11- or 2-fluoro-6, 12-dichloro-tetracene, ora mixture thereof, at elevated temperature with selenium to 2-fluoro-5, 6, 11, 12-tetraselenotetracene.

10. Use of a compound of the formula according to claim 1 as organic conductor element.

**Revendications**

1. Complexe de formule I:

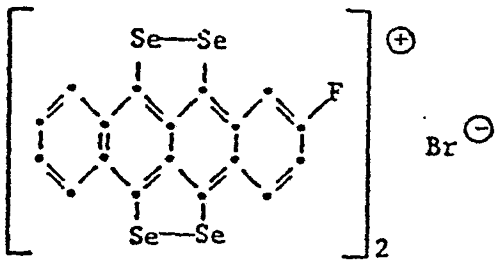 (I).

2. Procédé pour préparer le complexe de formule I selon la revendication 1, selon lequel on oxyde le fluoro-2 tétraséléno-5, 6, 11, 12 tétracène par le brome ou par un sel de brome oxydant libérant un bromure, en présence d'un solvant organique inerte.

3. Procédé pour préparer le complexe de formule I selon la revendication 1, selon lequel on co-sublime le fluoro-2 tétraséléno-5, 6, 11, 12 tétracène et le brome.

4. Procédé pour préparer le complexe de formule I selon la revendication 1, selon lequel on oxyde électrochimiquement le fluoro-2 tétraséléno-5, 6, 11, 12 tétracène en présence d'un solvant organique inerte et d'un électrolyte conducteur renfermant un bromure.

5. Procédé selon la revendication 4 caractérisé en ce qu'on utilise, comme électrolyte conducteur, un composé répondant à la formule II:

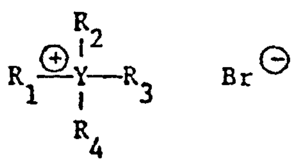 (II)

dans laquelle X représente N, P ou As tandis que $R_1$ à $R_4$ représentent chacun, indépendamment les uns des autres, un alkyle en $C_1$-$C_{18}$, un benzyle, un phényle ou un naphtyle.

6. Procédé selon la revendication 5 caractérisé en ce qu'on utilise un composé de formule II dans lequel Y représente N ou p, $R_1$ représente un radical benzyle ou phényle, et $R_2$ à $R_4$ représentent chacun un radical alkyle linéaire contenant de 1 à 12 atomes de carbone ou un radical phényle, ou encore $R_1$ à $R_4$ représentent chacun un radical alkyle linéaire contenant de 1 à 12 atomes de carbone.

7. Procédé selon la revendication 5 caractérisé en ce qu'on utilise un composé de formule II dans lequel Y représente N tandis que $R_1$ à $R_4$ représentent chacun un radical alkyle linéaire contenant de 1 à 6 atomes de carbone, plus particulièrement chacun un radical n-butyle.

8. Composés répondant à l'une des formules III à VI:

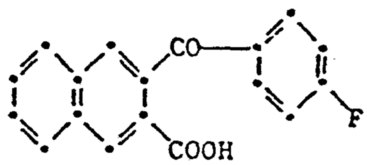

(III),

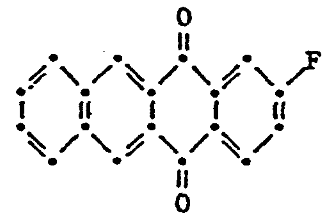

(IV),

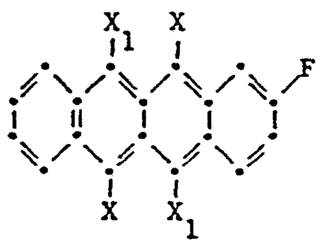

(V)

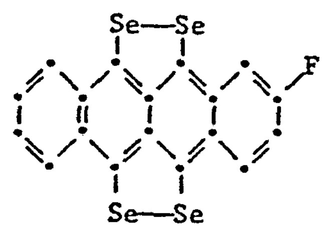

(VI),

dans lesquelles les $X_1$ et les X représentent chacun l'hydrogène, ou les $X_1$ représentent chacun l'hydrogène et les X chacun le chlore, ou les $X_1$ représentent chacun le chlore et les X chacun l'hydrogène.

9. Procédé pour préparer les composés de formules III à VI selon la revendication 8, procédé caractérisé en ce qu'on fait réagir l'anhydride de l'acide naphtalène-dicarboxylique-2,3 en présence d'un catalyseur de Friedel et Crafts avec le fluorobenzène pour obtenir le composé de formule III, on cyclise l'acide (fluoro-4 benzoyl)-2 naphtalène-carboxylique-3 de manière à le convertir en la fluoro-2 naphtacène-quinone-5, 12 de formule IV, on convertit celle-ci, par réduction, en le fluoro-2 tétracène (composé de formule V dans lequel $X_1$ et X représentent H), on fait réagir le chlorure de fluoro-2 tétracène avec le chlorure de sulfuryle, réaction qui conduit au fluoro-2 dichloro-5, 11 tétracène ou au fluoro-2 dichloro-6,12 tétracène, et ensuite on fait réagir le fluoro-2 dichloro-5,11 tétracène ou le fluo-ro-2 dichloro-6,12 tétracène, ou un mélange de ceux-ci, à température élevée, avec le sélénium pour obtenir le fluoro-2 tétraséléno-5, 6, 11, 12 tétracène.

10. Application du composé de formule I selon la revendication 1 comme élément conducteur organique.

Fig. 1

PROJEKTION DER KRISTALLSTRUKTUR AUF DIE (a,b)-EBENE

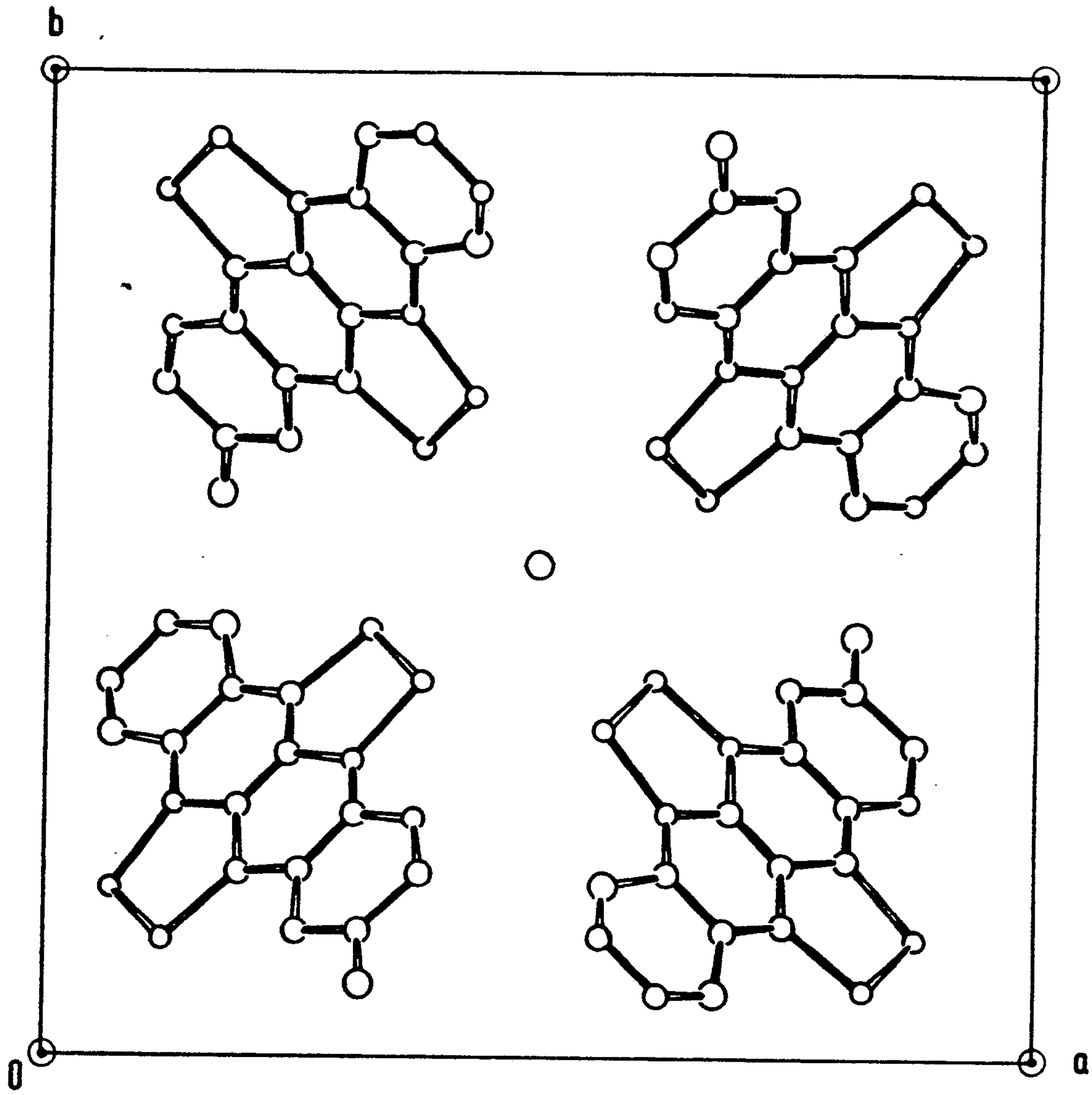

Fig. 2

SPEZIFISCHER ELEKTRISCHER WIDERSTAND NORMIERT AUF 295 K
IN ABHÄNGIGKEIT VON DER TEMPERATUR VON $(FTSeT)_2Br$